# EUROPEAN PATENT SPECIFICATION

(11) **EP 2 228 044 B1**
(45) Date of publication and mention of the grant of the patent: **06.01.2016**
(21) Application number: 10002345.6
(22) Date of filing: 08.03.2010
(51) Int. Cl.: A61F 13/534

(54) **Disposable towelette for genito-urinary and surrounding area**
Wegwerftuch für die Urogenitalgegend und umliegende Regionen
Lingette jetable pour zones génito-urinaires et environnantes

(30) Priority: 09.03.2009 IT CZ20090004
(43) Date of publication of application: 15.09.2010
(73) Proprietor: Lagano, Rocco, 88024 Girifalco (CZ) (IT)
(72) Inventor: Lagano, Rocco, 88024 Girifalco (CZ) (IT)
(74) Representative: Garavelli, Paolo

(56) References cited:
- WO-A1-2007/114744
- GB-A- 836 395

## Description

The present invention refers to a disposable towelette for genito-urinary and surrounding areas.

Referring to the field of infections in the genito-urinary and surrounding areas, current surveys have demonstrated the widespread occurrence of infections in these areas, which must be prevented, and which currently are not efficiently solved by currently-marketed cotton towelettes.

In fact, current surveys have demonstrated that over 40% of nocosomial infections are care of the genito-urinary apparatus, even in the most developed industrial countries. In order to oppose the direct contagion, physicians in affected fields (urology, nephrology, gynecology, dermathology, general medicine) can only give general advices, since they have no instruments for preventing the direct infection (for example the promiscuous use of a towel) for the simple reason that nowadays on the market there are no disposable towelettes that can be individually used only once, in order to be able to remove the main vehicle of an indirect contagion among people living in the same environments. G-B-A-836 395 discloses a napkin composed of two different layers, one of which is placed whodly around the other.

Common cotton towelettes, in addition to be vehicles of infection (bacteria, viruses and micetes live in their wefts) imply other disadvantages:
- they do not ensure the perfect drying of the part, due to their scarce absorbance;
- the worked tissue can generate, when drying, microlesions which favour the occurrence of infections.

Object of the present invention is solving the above prior-art problems, by providing a disposable towelette that can be used for personal hygiene and prevention of infections in the genito-urinary and surrounding areas; such towelette has low production costs, is composed of easily obtainable materials and can be produced using common machines for producing disposable products.

The above and other objects and advantages of the invention, as will appear from the following description, are obtained by a disposable towelette as claimed in Claim 1. Preferred embodiments and non-trivial variations of the present invention are claimed in the dependent Claims.

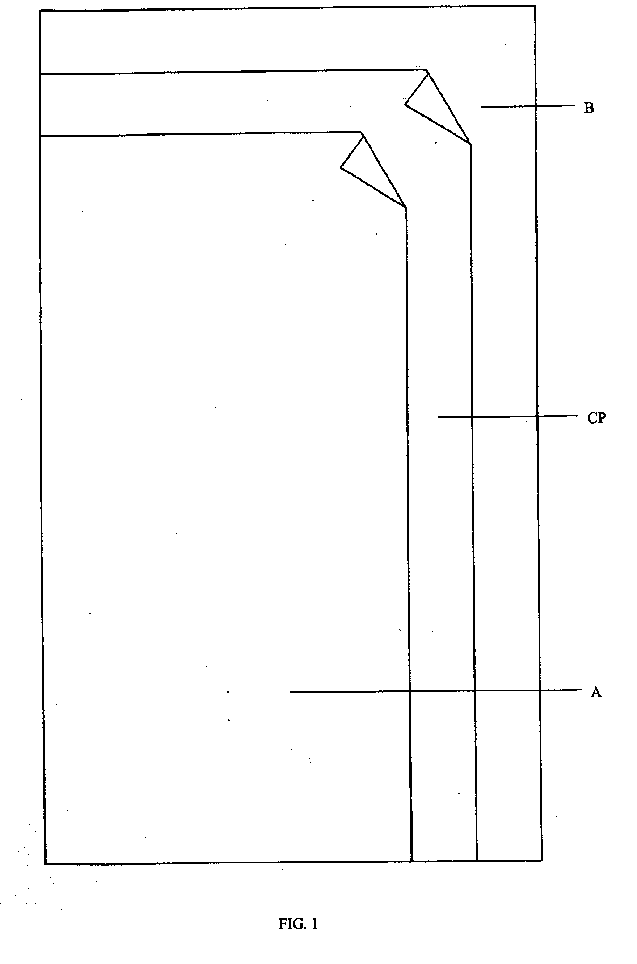

The present invention will be better described by some preferred embodiments thereof, given as a non-limiting example, with reference to the enclosed drawings, in which the only Figure 1 shows a side schematic view of a preferred embodiment of the towelette of the invention.

With reference to Fig. 1, the disposable towelette of the invention comprises:
- a first layer A made of soft, semi-waterproof viscose (preferably 2 mm thick) to guarantee a soft, pleasant sensation when used to cleanse the genito-urinal and anal areas. The semi-waterproof viscose allows the passage of water polluted by micro-organisms towards the central part and prevents its return back to the surface;
- a second layer or central part CP, that is made up of highly-absorbent non-woven tissue to guarantee the capturing of every single drop of water quickly and efficiently, ensuring a perfectly dry surface where used; and
- a third layer B equal to the first layer A and made of the same material.

In the arrangement shown in Figure 1, the second layer CP is sandwiched between the first layer A and the third layer B.

The above three layers are mutually heat-sealed in order to produce the towelette of the invention.

The preferred sizes of this towelette are 25 x 45 cm.

With the above towelette it is possible to prevent primary infections caused by the indirect contact with the genito-urinary and surrounding areas. The heat-sealing of the three overlapping layers was studied to make the disposable towelette of the invention resistant to rubbing and to humidity. Its disposability guarantees against any germ contamination.

The use of a common cotton towelette, used more than once, can become a vehicle or agent of infection, whereas the disposable towelette of the invention is disposed of with all its impurities.

Purpose of the particular structure of this towelette is perfectly drying the treated part, so that, in the covered body parts, no hot-humid environments are created, which favour the growth of microorganisms that can damage the health.

The triple-layer nature of the towelette is realised as follows: the two external layers A and B are made of soft viscose and, in addition to being very comfortable upon contact with the skin, can be quickly crossed by water polluted by possible patogen microorganisms, that can be found deposited also on the skin and genitals after their washing. The complete and quick absorption occurs because the central part CP is thicker (5 mm) than the first and second layers A, B (2 mm). The central part CP then operates as water catalyst and, after having absorbed all residual humidity particles on skin and genitals, tends to keep these particles (due to its greater consistency) without making them go back to the surface.

The end result is double: a perfectly dry skin (removal of the hot-humid environment that favours the growth of germs and micetes), and the final removal of all infecting agents captured by the towelette.

The main characteristics of the towelette of the invention are:
- high absorption capacity: 450% with an absorption time of 0.70 seconds;
- high resistance to humidity and rubbing;
- it does not cause microlesions to treated parts, since its components are smooth and soft.

The towelette of the invention can be easily used as follows:
1) washing the affected parts (those having an infection or only as daily hygiene, particularly for women during their menstrual cycles) according to physician's prescriptions and protocols;
2) drying with the 3-layered towelette of the invention;
3) disposing the used towelette.

## Claims

1. Disposable towelette for genito-urinary and surrounding areas, comprising:
- a first layer (A) made of soft, semi-waterproof viscose, the semi-waterproof viscose having such a pore structure as to be adapted to allow the passage of water polluted by micro-organisms towards the central part (CP) and preventing its return back to the surface, said first layer (A) having a first thickness;
- a second layer or central part (CP), made up of absorbing non-woven tissue adapted to guarantee the capturing of every single drop of water to ensure a dry surface where used, said second layer (CP) having a second thickness; and
- a third layer (B) equal to the first layer (A) and made of the same material, said third layer (B) having thereby the first thickness, namely the same thickness of said first layer (A),
wherein the second layer (CP) is sandwiched between the first layer (A) and the third layer (B),
**characterised in that**:
- the first layer (A), the second layer (CP) and the third layer (B) are three separate layers which, when manufacturing the disposable towelette, are stacked and joined by heat-sealing one to the other; and
- the second thickness is more than double with respect to the first thickness.

2. Disposable towelette according to claim 1, wherein the sizes of the towelette are 25 x 45 cm.

3. Disposable towelette according to claim 1, wherein the first layer (A) and the third layer (B) of the towelette are 2 mm thick.

4. Disposable towelette according to claim 1, wherein the second layer or central part (CP) is 5 mm thick.

## Patentansprüche

1. Einwegtuch für den Urogenital-Bereich und die umliegenden Bereiche, das folgendes einschließt:
- eine erste Schicht (A) aus semipermeabler weicher Viskose, die semipermeable Viskose hat eine poröse Struktur, die den Durchfluss von durch Mikroorganismen verschmutztem Wasser zum Zentralbereich (CP) ermöglicht und die erneute Rückkehr an die Oberfläche verhindert, die genannte erste Schicht (A) hat eine erste Dicke;
- eine zweite Schicht oder Zentralbereich (CP) aus nicht absorbierendem Gewebe, das dazu dient, das Auffangen jedes einzelnen Wassertropfens zu garantieren, um eine trockene Oberfläche während des Gebrauchs zu garantieren, die genannte zweite Schicht (CP) hat eine zweite Dicke; und
- eine dritte Schicht (B), die der ersten Schicht (A) entspricht und aus dem gleichen Material besteht, die genannte dritte Schicht (B) hat auf diese Weise die erste Dicke, d. h. die gleiche Dicke der genannten ersten Schicht (A),
in der die zweite Schicht (CP) zwischen der ersten Schicht (A) und der dritten Schicht (B) eingefügt ist,
und **dadurch gekennzeichnet ist, dass**:
- die erste Schicht (A), die zweite Schicht (CP) und die dritte Schicht (B) drei separate Schichten sind, die, wenn das Einwegtuch hergestellt wird, aufeinander geschichtet und miteinander durch Thermoversiegelung vereint werden; und
- die zweite Schicht doppelt so dick wie die erste Schicht ist.

2. Einwegtuch gemäß Patentanspruch 1, dessen Abmessungen 25 x 45 cm entsprechen.

3. Einwegtuch gemäß Patentanspruch 1, in dem die erste Schicht (A) und die dritte Schicht (B) des Tuches 2 mm dick sind.

4. Einwegtuch gemäß Patentanspruch 1, dessen zweite Schicht oder Zentralbereich (CP) 5 mm dick ist.

## Revendications

1. Serviette jetable pour zones génito-urinaires et environnantes comprenant :
- une première couche (A) en viscose semi-imperméable souple dont la structure poreuse permet le passage d'eau, polluée par des micro-organismes, vers la partie centrale (CP) et empêche le retour à la surface ; cette première couche (A) a une première épaisseur ;
- une seconde couche ou partie centrale (CP), en textile non tissé absorbant qui capture chaque goutte d'eau pour garantir une surface sèche pendant l'utilisation ; cette seconde couche (CP) a une seconde épaisseur ; et
- une troisième couche (B) identique à la première couche (A) composée de la même matière que celle-ci ; cette troisième couche (B) a la même épaisseur que la première couche (A),
où la seconde couche (CP) est intercalée entre la première couche (A) et la troisième couche (B),
**caractérisée en ce que** :
- la première couche (A), la seconde couche (CP) et la troisième couche (B) sont trois couches séparées qui, lors de la préparation de la serviette jetable, sont empilées et unies, l'une sur l'autre, par scellement thermique ; et
- la seconde épaisseur est plus que le double de la première épaisseur.

2. Serviette jetable, selon la revendication 1, dont les dimensions de la serviette sont égales à 25 x 45 cm.

3. Serviette jetable, selon la revendication 1, dont la première couche (A) et la troisième couche (B) de la serviette ont une épaisseur de 2 mm.

4. Serviette jetable, selon la revendication 1, où la seconde couche, à savoir la partie centrale (CP), a une épaisseur de 5 mm.
